# EUROPEAN PATENT SPECIFICATION

(11) **EP 3 488 850 B1**
(45) Date of publication and mention of the grant of the patent: **19.06.2024**
(21) Application number: 17833256.5
(22) Date of filing: 20.04.2017
(51) Int. Cl.: A61K 31/496, A61P 35/02

(54) **NOVEL APPLICATION OF GZD824 AND PHARMACEUTICALLY ACCEPTABLE SALTS THEREOF IN TREATING DISEASES**
NEUARTIGE VERWENDUNG VON GZD824 UND PHARMAZEUTISCH AKZEPTABLE SALZE DAVON ZUR BEHANDLUNG VON KRANKHEITEN
NOUVELLE APPLICATION DE GZD824 DANS LE TRAITEMENT DE LA LEUCÉMIE LYMPHOBLASTIQUE.

(30) Priority: 25.07.2016 CN 201610592371; 11.11.2016 CN 201611040298
(43) Date of publication of application: 29.05.2019
(73) Proprietor: HEALTHQUEST PHARMA INC., Guangzhou, Guangdong 510530 (CN)
(72) Inventor: LI, Peng, Guangzhou Guangdong 510530 (CN); YE, Wei, Guangzhou Guangdong 510530 (CN); DING, Ke, Guangzhou Guangdong 510530 (CN); LU, Xiaoyun, Guangzhou Guangdong 510530 (CN)
(74) Representative: Marks & Clerk LLP
(86) International application number: PCT/CN2017/081173
(87) International publication number: WO 2018/018950

(56) References cited:
- EP-A1- 2 594 567
- CN-A- 101 885 722
- CN-A- 103 539 784
- XIAOMEI REN ET AL: "Identification of GZD824 as an Orally Bioavailable Inhibitor That Targets Phosphorylated and Nonphosphorylated Breakpoint Cluster Region-Abelson (Bcr-Abl) Kinase and Overcomes Clinically Acquired Mutation-Induced Resistance against Imatinib", JOURNAL OF MEDICINAL CHEMISTRY, vol. 56, no. 3, 14 February 2013 (2013-02-14), pages 879-894, XP055099026, ISSN: 0022-2623, DOI: 10.1021/jm301581y

## Description

### TECHNICAL FIELD

The present invention belongs to medical biology, specifically relates to (3-((1H-pyrazol[3,4-b]pyridine-5-substituted)ethinyl)-4-methyl-N-(4-((4-methylpiperazine-1-substituted)methyl)-3-(trifluorome thyl)phenyl)benzamide) (Nectinib or GZD824) and a pharmaceutically acceptable salt thereof for a novel use in treatment.

### BACKGROUND OF THE INVENTION

Acute lymphoblastic leukemia, ALL, is a malignant neoplastic disease originated from the intramedullary abnormal hyperplasia of B or T lymphocyte. Abnormal hyperplasia blast cells can aggregate in the marrow and inhibit the normal hematopoietic function while invading extramedullary tissues such as meninges, lymph nodes, gonads, and liver etc. In China, ALL incidence rate is about 0.67/100,000, wherein the incidence rate in oil fields and polluted area is prominently higher than that across the nation. The peak of incidence for ALL is during the childhood (0~9 years old) and it can account for over 70% of child leukemia. ALL accounts for about 20% of adult leukemia in adult patients and it belongs to the high risk leukemia. Acute lymphoblastic leukemia can be divided into B cell type and T cell type based on the immune markers, wherein the former can be divided into four subtypes, i.e. Null-ALL, Common-ALL, Pre-B-ALL and B-ALL.

Precursor B-cell lymphoblastic leukemia (pre-B ALL) is a form of ALL, which characterizes in that early B lymphocyte precursor malignantly proliferates and aggregates in marrow, blood and lymphoid organs. It is also the most common type of ALL. At present, clinic treatments for pre-B ALL are mainly chemotherapy, which presents a high recurrence rate and occurs in about 25% children. Treatments with non-specific antineoplastic drugs can cause severe toxic side effect. Therefore, the development for novel drugs in treating precursor B-cell acute lymphocyte has been extremely important and urgent.

Non-receptor tyrosine kinase Src belongs to members of highly conserved Src-family protein tyrosine kinases. Src family is the earliest and most deeply studied family including members such as Blk, Brk, Fgr, Frk, Fyn, Hck, Lck, Lyn, c-Src, Srm and c-Yes. According to the amino acid sequences, it can be divided into two subfamilies, one of them includes Src, Fyn, Yes and Fgr which are widely expressed in different tissues; the other one of them includes Lck, Blk, Lyn and Hck which are related to hematopoietic cells. SCR has high expression in multiple human tumors such as breast cancer, lung cancer, melanoma, adenocarcinoma, head and neck cancer, ovarian cancer, colon cancer cells and leukemia, and its activity is closely related to the occurrence, development and prognosis of tumors.

Although the joint use of Imatinib in the treatment of ALL during the induction and consolidation phases obtains very good effect, central nervous system leukemia (CNS-L) can still easily occur during the treatment process. Besides, although Imatinib can increase the CR rate of Ph-ALL, it cannot prominently increase the survival rate. Studies show that BCR-ABL kinase mutation occurs in Ph-ALL patients before the treatment and the situation of Src kinase activation also exists. Therefore, the search for ABL/SRC double kinase inhibitor is the most promising ALL treatment strategy.

(3-((1H-pyrazol[3,4-b]pyridine-5-substituted)ethinyl)-4-methyl-N-(4-(( 4-methylpiperazine-1-substituted)methyl)-3-(trifluoromethyl)phenyl)be nzamide)(Nectinib, GZD824 for acronym) is a type of third generation small molecule inhibitor mutated from Bcr-Abl^{WT} and Bcr-Abl^{T315I}, which can effectively treat chronic myeloid leukemia and particularly presents better treatment effect on anti-leukemia patients resistant to Gleevec [CN201010216603.7; J.Med.Chem.2013].

Xiaomei Ren et al., in "Identification of GZD824 as an Orally Bioavailable Inhibitor That Targets Phosphorylated and Nonphosphorylated Breakpoint Cluster Region-Abelson (Bcr-Abl) Kinase and Overcomes Clinically Acquired Mutation-Induced Resistance against Imatinib", J. Med. Chem., 2013, 56, 879-894, report GZD824 as a novel orally bioavailable inhibitor against a broad spectrum of Bcr-Abl mutants. It is reported that the compound suppresses proliferation of Bcr-Abl-positive K562 and Ku812 human CML cells.

In EP 2594567 A1 (Guangzhou Institute of Biomedicine And Health), specific heterocyclic alkynyl benzene compounds, their pharmaceutically acceptable salts and stereoisomers thereof are described, including their application in preparing drugs for preventing or treating tumors. It is described that the compounds can overcome clinically induced resistance against Gleevec.

However, whether (3-((1H-pyrazol[3,4-b]pyridine-5-substituted) ethinyl)-4-methyl-N-(4-((4-methylpiperazine-1-sub stituted)methyl)-3-(t rifluoromethyl)phenyl)benzamide) (GZD824 for acronym) can have an excellent therapeutic effect on Ph-negative precursor B-cell lymphoblastic leukemia (pre-B ALL), is still unknown yet.

### SUMMARY OF THE INVENTION

Based on this, one of the aims of the present invention is to provide (3-((1H-pyrazol[3,4-b]pyridine-5-substituted)ethinyl)-4-methyl-N-(4-((4-methylpiperazine-1-substituted)methyl)-3-(trifluoro methyl)phenyl)benzamide) (Nectinib, GZD824 for acronym) and its pharmaceutically acceptable salt for a novel use in treating diseases.

Technical solutions for achieving the above-mentioned aim are as follows.

(3-((1H-pyrazol[3,4-b]pyridine-5-substituted)ethinyl)-4-methyl-N-(4-((4-methylpiperazine-1-substituted)methyl)-3-(trifluoromethyl)ph enyl)benzamide) and a pharmaceutically acceptable salt thereof for use in a method of treating Ph-negative precursor B-cell lymphoblastic leukemia.

In the present invention, the inventors inventively discovered GZD824 (Nectinib) for the novel use with long-term experiments and from thousands of similar compounds. GZD824 can, by targeting SRC protein kinase, be applied in the treatment of acute lymphocytic leukemia, particularly in the treatment of precursor B-cell lymphoblastic leukemia, and particularly in the treatment of Ph-negative precursor B-cell lymphoblastic leukemia.

### BRIEF DESCRIPTION OF THE DRAWINGS

Fig. 1 is the diagram of cell activity test results by treating pre-B ALL cell line (NALM 6 and SUPB15) with GZD824 of different concentration respectively.
Fig. 2 is the diagram of the apoptosis test results after treating NALM6 and SUPB15 cells with GZD824 (3µM, 1µM) and DMSO respectively.
Fig. 3 is the diagram of the cell cycle test results of treating NALM6 and SUPB15 cells with GZD824 (3µM) and DMSO respectively.
Fig. 4 is the diagram of the apoptosis test results of treating primary pre-B ALL cells of five patients with GZD824 (1µM) and DMSO respectively.
Fig. 5 is the diagram of the apoptosis test results of co-culturing B cells of healthy donators with GZD824 (1µM) or DMSO.
Fig. 6 is the diagram of results of treating PDX mouse model with Imatinib and DMSO.
Fig. 7 is the diagram of results of treating PDX mouse model constructed in the immunodeficient mouse (NSI) with GZD824, Imatinib and DMSO respectively for two weeks.
Fig. 8 is the diagram of results of ratios of the detected Pre-B ALL in blood, spleen and marrow of mouse after treating PDX mouse constructed with primary pre-B ALL originated from patient P#1 with GZD824, Imatinib and DMSO.
Fig. 9 is the diagram of results of ratios of the detected Pre-B-ALL in blood, spleen and marrow of mouse by flow cytometry after treating PDX mouse constructed with primary pre-B ALL originated from patient P#1 with GZD824, Imatinib and DMSO for two weeks and then executed.
Fig. 10 is the diagram of results of ratios of human CD45 positive cells by statistical analysis of blood, spleen and marrow of mouse after treating PDX mouse constructed with primary pre-B ALL originated from patients P#1(B), P#2(C) and P#3(D) with GZD824, Imatinib and DMSO.
Fig. 11 is the diagram of results of ratios of the detected pre-B ALL in blood, spleen and marrow of mouse after treating PDX mouse constructed with primary pre-B ALL originated from patient P#4 with GZD824, Imatinib and DMSO.
Fig. 12 is the diagram of results of ratios of human CD45 positive cells by statistical analysis of blood, spleen and marrow of mouse after treating PDX mouse constructed with primary pre-B ALL originated from patients P#4(F) and P#5(G) with GZD824, Imatinib and DMSO.
Fig. 13 is the diagram of results of detected NALM6 cells treated with GZD824 by Western blot method.
Fig. 14 is the diagram of results of detected expressions of four genes, i.e. BCL-XL, CCND1, CDK4 and CCKN1A after treating NALM6 cells with 3µM GZD824 or DMSO for 24 hours.
Fig. 15 is the diagram of results of detected apoptosis with apoptosis kit (ANNEXIN V, PI) after treating NALM6 cell with GZD824, SRC inhibitor (KX2-391), STAT3 inhibitor (HO-3867) or DMSO for 24 hours.
Fig. 16 is the diagram of results of ratios of ANNEXIN V positive cells by statistical analysis in Example 6.
Fig. 17 is the diagram of results of ratios of ANNEXIN V positive cells by statistical analysis.
Fig. 18 is the diagram of results of detected NALM6 cells treated with GZD824 by Western blot method.
Fig. 19 is the diagram of results of detected primary pre-B ALL cells treated with GZD824 by Western blot method.
Fig. 20 is the diagram of mechanism of GZD824 killing leukemia cells.

### DETAILED DESCRIPTION OF PREFERRED EMBODIMENTS

After long-term experiments, inventors of the present invention discovered that GZD824 (Nectinib) has functions on decreasing pre-B ALL cell activity, inducing pre-B ALL cell cycle arrest, and inducing pre-B ALL apoptosis. Besides, Ph-pre-B ALL cells are more sensitive to GZD824 than Ph+pre-B ALL cells. GZD824 can also down-regulate phosphorylation of SRC kinase, STAT3, RB and c-Myc.

(3-((1H-pyrazol[3,4-b]pyridine-5-substituted)ethinyl)-4-methyl-N-(4-(( 4-methylpiperazine-1-substituted)methyl)-3-(trifluoromethyl)phenyl)be nzamide), hereinafter referred to as GZD824 or D824 in the figures, was synthesized and obtained in accordance with known methods.

### Example 1

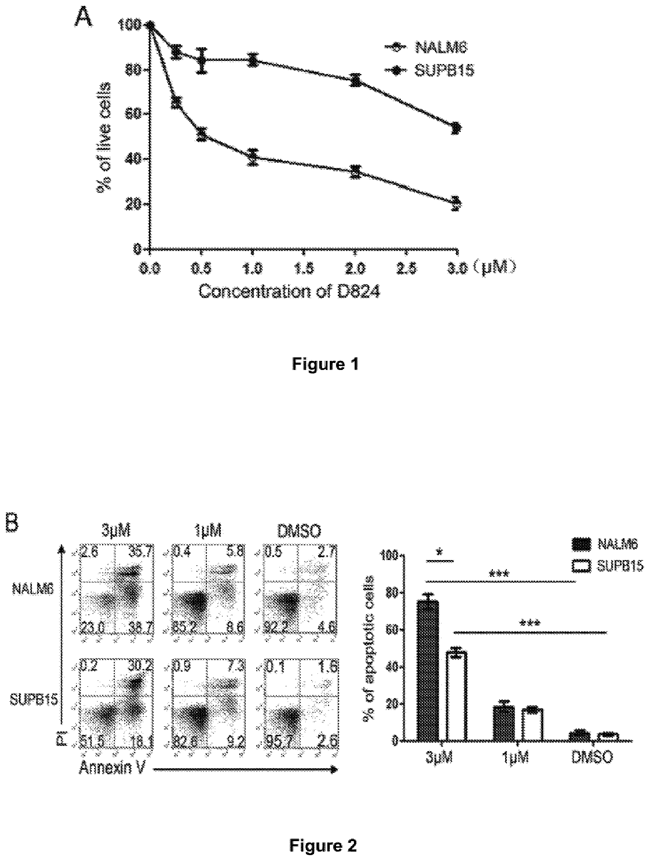

GZD824 has better anti-tumor effect on acute lymphoblastic leukemia, particularly on precursor B-cell lymphoblastic leukemia. GZD824 can suppress the proliferation of pre-B ALL cell line, induce its apoptosis, and suppress the pre-B ALL cell cycle at G0/G1 phase.
1.1 Two types of cells, i.e. NALM6 (Ph-negative acute lymphoblastic leukemia cell line) and SUPB15 (Ph-positive acute lymphoblastic leukemia cell line, reference example), were treated with GZD824 of different concentrations (0-3 µM/L) respectively. After 24 hours, CCK8 is added. The mixture was incubated for 2 hours, and then its absorption value at 450nm was detected with a microplate reader. Results show that the treatment with GZD824 can prominently decrease the cell activity thereof, demonstrating that GZD824 can prominently suppress the proliferation of the above-mentioned two types of cells, particularly inhibit the proliferation of NALM6 and the inhibition rate is positively related to the concentrations of drugs. We plotted a chart of cell activity based on the growth inhibition function of GZD824 on these six types of cells, which is as shown in Fig. 1.
1.2 After treating NALM6 and SUPB15 cells respectively with GZD824 (3µM, 1µM) and DMSO of different concentrations for 24 hours, apoptosis was detected using an apoptosis kit (ANNEXIN V, PI). Please refer to Fig. 2 for the results. After treating the two types of cells NALM6 and SUPB15 respectively with GZD824 of different concentrations (3 µM/L, 1 µM/L) for 24 hours, the cells were stained with the apoptosis kit (ANNEXIN V, PI), and apoptosis was detected with flow cytometry. The results are shown in Fig. 2.

Results show that the treatment with GZD824 can prominently enhance apoptosis of these two types of cells, demonstrating that GZD824 can prominently facilitate the apoptosis of the above-mentioned two types of cells, particularly enhance the apoptosis of NALM6 cells. The left side of Fig. 2 shows the flow pattern of apoptosis after treatment with GZD824 and DMSO; the right side of Fig. 2 shows the ratio of ANNEXIN V positive cells, through statistical analysis, after treatment with GZD824 and DMSO.

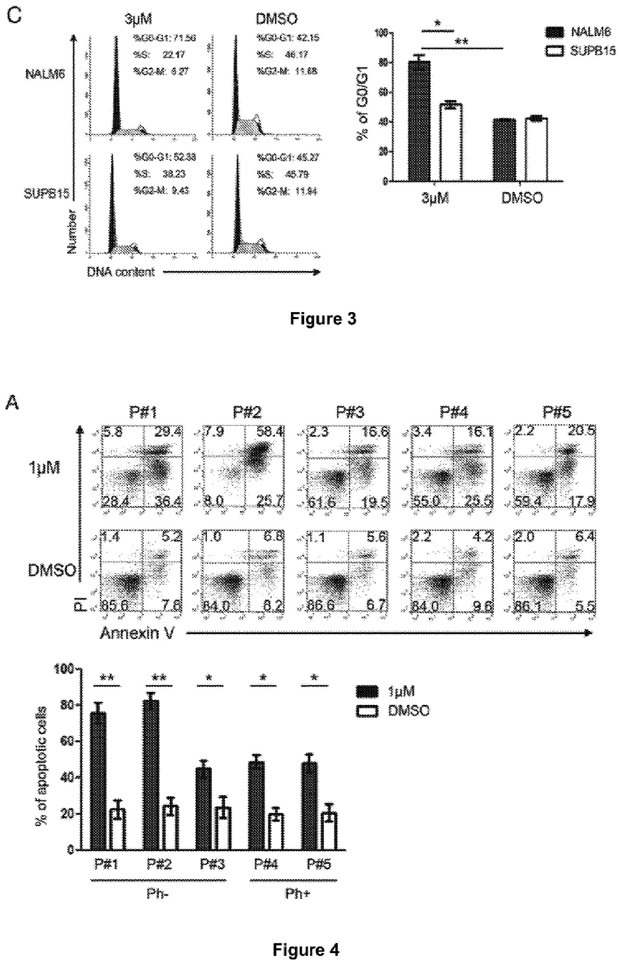

1.3 After treating the two types of cells, i.e. NALM6 and SUPB15, with GZD824 (3 µM/L) and DMSO respectively for 24 hours and then treating them with a cell cycle kit, the cell cycle was measured with flow cytometry. The results show that treatment with GZD824 can increase the ratio of these two types of cells at G0/G1 phase, demonstrating that GZD824 can suppress the cell cycle of the above-mentioned two types of cells at G0/G1 phase, particularly suppressing the cell cycle of NALM6 cells. Please refer to Fig. 3 for the results. The left side of the figure shows the flow pattern of the treated cell cycle; the right side shows the ratios of the treated ANNEXIN V positive cells by statistical analysis.

### Example 2

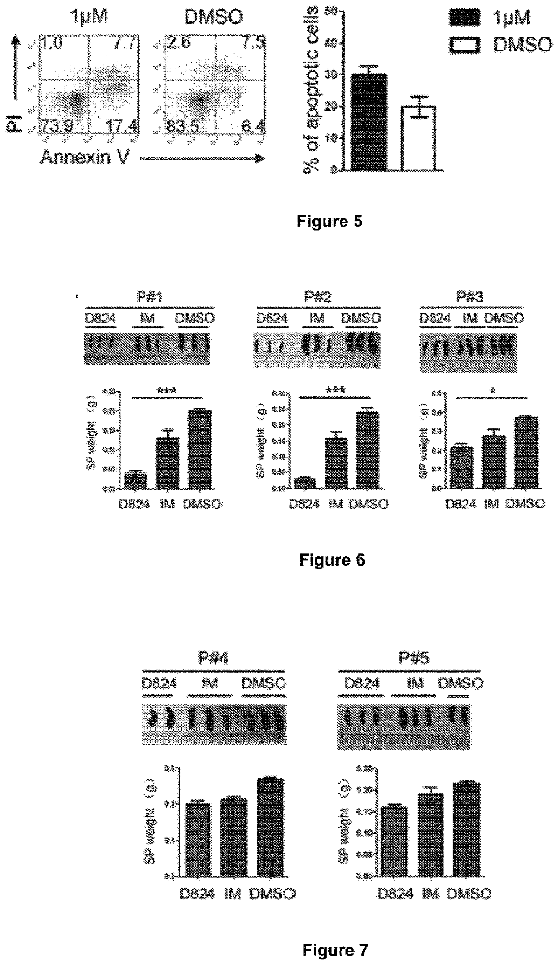

Experiments show that GZD824 can accelerate the cell apoptosis of the primary pre-B ALL cells from patients without killing B cells.
2.1 Marrow puncture samples from acute lymphoblastic leukemia patients were separated with lymphocyte separation fluid so as to separate leukemia cells. P#1, P#2, P#3 acute lymphoblastic leukemia cells are originated from three Ph- patients, and P#4 , P#5 are originated from two Ph+ patients (reference examples). After treating the leukemia cells from the five patients with GZD824 (1 µM/L) for 24 hours and staining them with an apoptosis kit (ANNEXIN V, PI), apoptosis was detected with flow cytometry. Results show that treatments with GZD824 can prominently enhance the apoptosis of leukemia cells, demonstrating that GZD824 can facilitate the apoptosis of leukemia cells from acute lymphoblastic leukemia patients until the leukemia cells are killed. Please refer to Fig. 4 for the results. Upper part of Fig. 4 shows the flow pattern of apoptosis of the primary cells treated with GZD824 and DMSO, the lower part of Fig. 4 shows the ratio of ANNEXIN V positive cells, by statistical analysis, treated with GZD824 and DMSO.
2.2 Killing of B cells by GZD824. Marrow puncture sample from normal donator was separated with lymphocyte separation fluid to obtain normal leucocytes, which are separated with magnetic beads to obtain the normal B cells. After co-culturing GZD824 (1 µM/L) with B cells from a healthy donor for 24 hours, the apoptosis is detected with an apoptosis kit (ANNEXIN V, PI). Results show that treatments with GZD824 cannot prominently facilitate the apoptosis of the primary B cells. It can be demonstrated with Fig. 4 that GZD824 can specifically kill the primary leukemia cells but does not kill the normal B cells. Please refer to Fig. 5 for the results Left side of Fig. 5 shows the flow chart of apoptosis treated with GZD824 and DMSO, right side of Fig. 5 shows the ratio of ANNEXIN V positive cells, by statistical analysis, treated with GZD824 and DMSO.

### Example 3

The present embodiment describes that GZD824 can suppress the proliferation of pre-B ALL cells in vivo in PDX mouse.
3.1 According to the conventional method, the primary leukemia cells from patients were transplanted to immunodeficient mouse (NSI) to construct PDX mouse model. Two months after the transplantation, leukemia cells from patients account for over 95% of the mouse spleen. 1×10⁶ leukemia cells originated from the spleen of the PDX mouse (P#1, P#2 and P#3) were transplanted through caudal vein to the NSI mouse bodies of eight weeks old and treated with a semilethal irradiation dose. The mice were divided into three groups with three mice in each group. When the ratio of human CD45 reaches 1%±0.2% of peripheral blood leukocyte of PDX mice by flow cytometry, the PDX mice were treated with GZD824 (25mg/kg), Imatinib (50mg/kg) and DMSO respectively for two weeks and then executed. By comparing the sizes of spleens of PDX mice administrated with GZD824, Imatinib and DMSO modeled in three patients, results show that the sizes of mouse spleens in the GZD824 group are prominently smaller than those of the other two groups, demonstrating that GZD824 can prominently reduce the infiltration of human leukemia cells (P#1, P#2, P#3) in mouse spleen. Please refer to Fig. 6 for the results. The upper figure shows the photos where the sizes of spleens from executed mice treated with GZD824, Imatinib and DMSO are compared; and the lower figure shows the weights of mouse spleens from GZD824, Imatinib and DMSO groups, by statistical analysis.
3.2 The primary leukemia cells from patients were transplanted to immunodeficient mouse (NSI) to construct PDX mouse model. 1×10⁶ leukemia cells originated from PDX mouse (P#4 and P#5, reference examples) were transplanted through caudal vein to the NSI mouse bodies of eight weeks old and treated with semilethal irradiation dose. The mice were divided into three groups with three mice in each group. When the ratio of human CD45 reaches 1%±0.2% of peripheral blood leukocyte of PDX mice by flow cytometry, the PDX mice were treated with GZD824, Imatinib and DMSO respectively for two weeks and then executed. By comparing the sizes of spleens of PDX mice administrated with GZD824, Imatinib and DMSO modeled in two patients, results show that the sizes of spleens in GZD824 group have no prominent difference from those of the other two groups, demonstrating that GZD824 cannot prominently reduce the infiltration of leukemia cells from the two patients (P#4 and P#5) on mouse spleen. Please refer to Fig. 7 for the results.

The upper part of Fig. 7 shows the photos where the sizes of spleens of the executed mice in GZD824, Imatinib and DMSO groups are compared.

The lower part of Fig. 7 shows the weights of mouse spleens from GZD824, Imatinib and DMSO groups by statistical analysis.

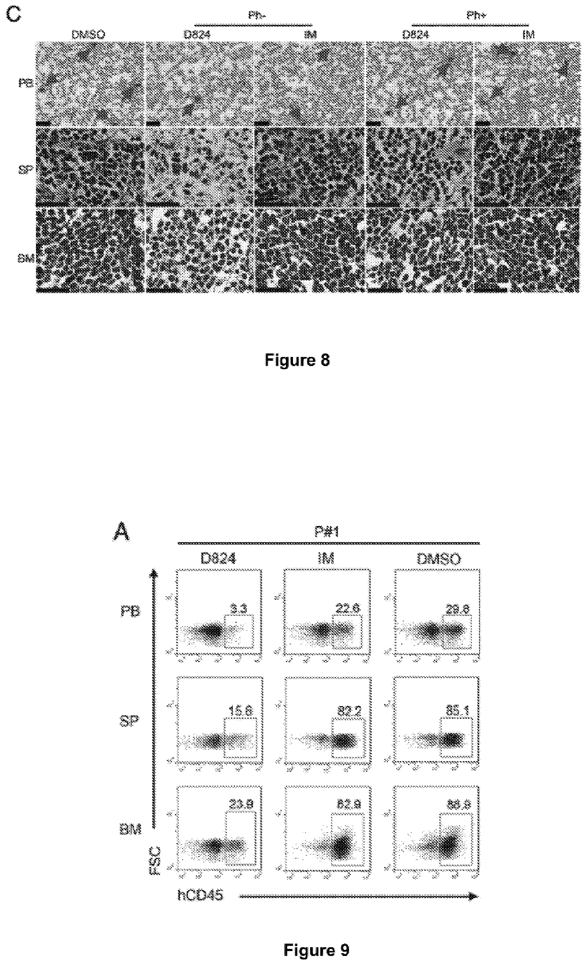

3.3 After executing mice in GZD824, Imatinib or DMSO groups, blood (upper Fig. 8), spleen (middle Fig. 8) and marrow (lower Fig.8) tissues were stained with Giemsa staining or Hematoxylin-eosin staining method. 10-20 µL of mouse blood drop was pipetted to a glass slide, and evenly spread on the slide, dried in shade at room temperature, stained with Giemsa working fluid for 15 minutes and eluted with eluents. After being fixed with neutral formaldehyde for 24 hours, the mouse spleen and marrow were dehydrated, made transparent, waxed, embedded, sliced, stained and then observed with a microscope. Results show that GZD824 can prominently decrease the infiltration of the primary leukemia cells originated from patient P#2 in mouse blood, spleen and marrow compared to those of Imatinib and DMSO.

### Example 4

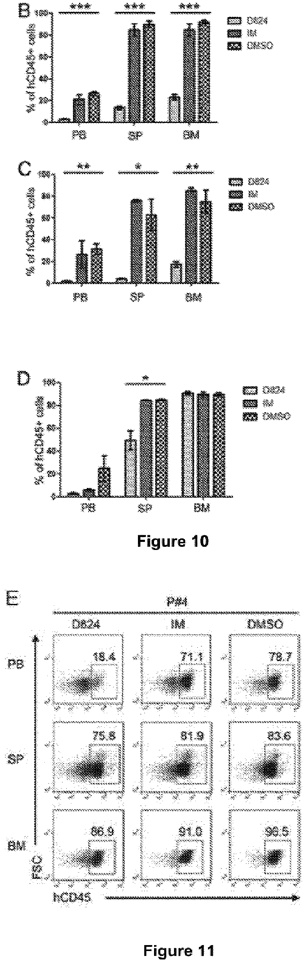

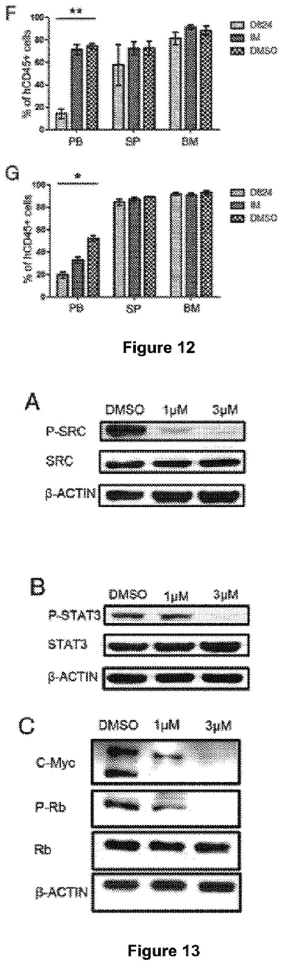

In the present embodiment, experimental researches on PDX mice constructed by the primary pre-B ALL of patient show that GZD824 can suppress the transplant rate of pre-B ALL.
4.1 After administration, PDX mice originated from patient P#1 were executed. The mouse blood was treated with red blood cell lysate to obtain the peripheral blood leukocytes. The mouse spleen was ground with 100 mesh screen to obtain spleen single cells. The marrow cells in the mouse marrow were washed with 1 mL injector to obtain mouse marrow single cells. The obtained mouse peripheral blood leukocytes, mouse spleen single cells and mouse marrow single cells from the above-mentioned steps were stained with anti-CD45-PE antibody and detected with flow cytometry. Results show that GZD824 can prominently decrease the ratio of human CD45 positive cells in mouse blood, spleen and marrow compared to those of Imatinib and DMSO, which demonstrates that GZD824 can prominently decrease the infiltration of the primary leukemia cells originated from patient P#1 in mouse blood, spleen and marrow compared to those of Imatinib and DMSO. Referring to Fig. 9 for the results, it shows the flow pattern of the ratio of human CD45 positive cells in mouse blood, spleen and marrow.
4.2 Using the same treatment method in Fig. 9, the ratios of CD45 positive cells in mouse blood, spleen and marrow were detected by flow cytometry and the ratios of CD45 positive cells in PDX mouse blood, spleen and marrow originated from different patients (P#1(B), P#2(C), P#3(D)) were obtained by statistical analysis. Results show that GZD824 can prominently decrease the ratios of human CD45 positive cells in mouse blood, spleen and marrow compared to those of Imatinib and DMSO, which demonstrates that GZD824 can prominently decrease the infiltration of the primary leukemia cells originated from patients P#1, P#2 and P#3 in mouse blood, spleen and marrow. Please refer to Fig. 10 for the results.
4.3 Using the same treatment method in Fig. 9, PDX mice originated from patient P#4 (reference examples) were executed after administration. The ratios of human CD45 positive cells in mouse blood, spleen and marrow were analyzed. Results show that GZD824 can prominently decrease the ratio of human CD45 positive cells in mouse blood compared to those of Imatinib and DMSO, which demonstrates that GZD824 can prominently decrease the infiltration of the primary leukemia cells originated from patient P#4 in mouse blood. Please refer to Fig. 11 for the results. It shows the flow chart of the ratios of human CD45 positive cells in mouse blood, spleen and marrow.
4.4 Using the same treatment method in Fig. 9, the ratios of CD45 positive cells in mouse blood, spleen and marrow were detected with flow cytometry and the ratios of CD45 positive cells in PDX mouse blood, spleen and marrow originated from different patients (P#4(F) and P#5(G), reference examples) were obtained by statistical analysis. Results show that GZD824 can prominently decrease the ratio of human CD45 positive cells in mouse blood compared to those of Imatinib and DMSO, which demonstrates that GZD824 can prominently reduce the infiltration of the primary leukemia cells originated from patients P#4 and P#5 in the mouse blood compared to those of Imatinib and DMSO. Please refer to Fig. 12 for the results.

### Example 5

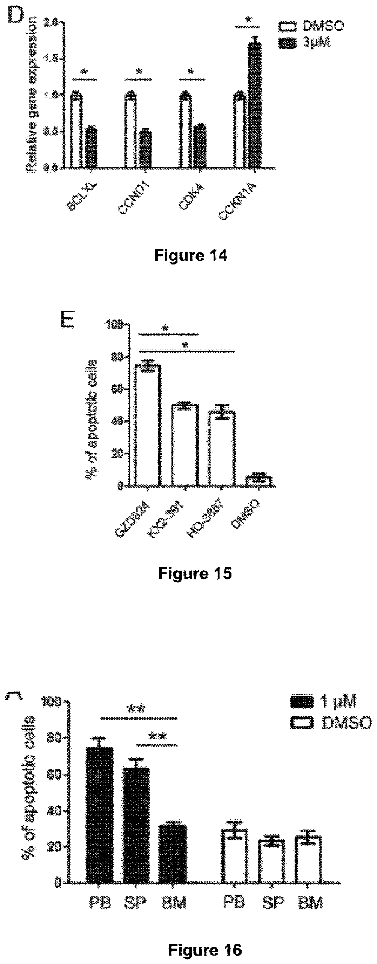

The present embodiment describes that GZD824 effectively suppresses the expression of phosphorylated SRC protein in pre-B ALL cells.
5.1. NALM6 cells treated with GZD824 were detected by Western blot method. After treating NALM6 cells with GZD824 of different concentrations (3 µM/L, 1 µM/L) for 24 hours, cells were collected and lysed to extract proteins. According to procedure of Western blot method, the extracted total proteins were analyzed with phospho-SRC, SRC, phospho-STAT3, STAT3, phospho-Rb, Rb and c-Myc antibodies. Results show that GZD824 suppresses the expression of phosphorylated SRC, phosphorylated STAT3 and phosphorylated Rb proteins in leukemia cells. From the protein perspective, GZD824 kills leukemia cells through suppressing SRC/STAT3 signal pathway. Please refer to Fig. 13 for the experimental results.
5.2 After being treated with 3 µM/L GZD824 and DMSO for 24 hours, NALM6 cells were collected to 1 ml TRIzol to extract RNA and reversely transcribed into cDNA. The expressions of four genes, i.e. BCL-XL, CCND1, CDK4 and CCKN1A, in these two groups of cells were detected with the fluorescence quantitative PCR method. Experimental results show that the expressions of BCL-XL, CCND1 and CDK4 genes are down-regulated and the expression of CCKN1A is up-regulated in NALM6 cells treated with GZD824 compared to the ones treated with DMSO, which means it can suppress the apoptosis, down-regulate the expressions of related genes facilitating cell cycles, and up-regulate the genes suppressing cell cycles. From the molecular perspective, the mechanism of GZD824 killing leukemia cells can be explained. For the experimental results, please refer to Fig. 14.
5.3 After treating NALM6 cells with GZD824, SRC suppressor (KX2-391), STAT3 suppressor (HO-3867) or DMSO for 24 hours, apoptosis was detected by using the apoptosis kit (ANNEXIN V, PI).

The ratio of ANNEXIN V positive cells was analyzed by statistical methods. Results show that the ratios of apoptosis in NALM6 cells treated with SRC suppressor (KX2-391) and STAT3 suppressor (HO-3867) are almost the same, demonstrating that GZD824 suppresses NALM6 cells through SRC/STAT3 signal pathway. Compared to SRC suppressor (KX2-391) and STAT3 suppressor (HO-3867), NALM6 cells treated with GZD824 show the highest ratio of apoptosis, demonstrating that GZD824 also kill the leukemia cells through suppression of other signal pathways apart from SRC/STAT3 signal pathway. For the experimental results, please refer to Fig. 15.

### Example 6

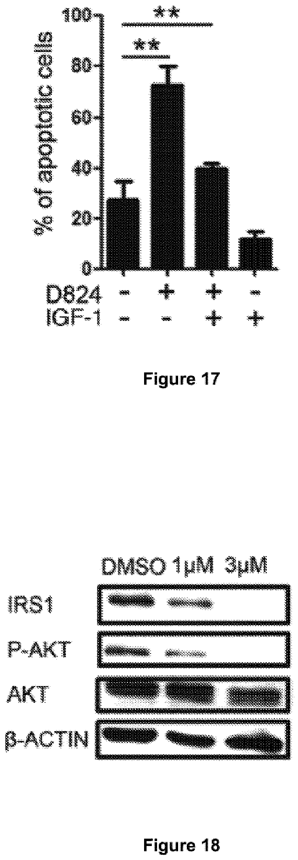

The present embodiment describes that GZD824 can effectively suppress the PI3K/AKT signal pathway in pre-B ALL cells.
6.1 Human primary leukemia cells were collected from P#2 PDX mouse blood, spleen and marrow and the leukemia cells originated from these three different mouse organs were co-cultured with 1 µM/L GZD824 or DMSO for 24 hours. The ratios of ANNEXIN V positive cells were analyzed by statistical methods. Results show that the ratio of apoptosis of leukemia cells originated from the mouse marrow is the lowest, demonstrating that the leukemia cells in the marrow environment possesses a lower sensitivity to GZD824 due to certain protective factor. Please refer to Fig. 16 for the results.
6.2 Human primary leukemia cells were extracted from P#1 PDX mouse spleen and co-cultured with DMSO, GZD824 (1 µM/L), IGF-1 (10 ng/mL), or GZD824 (1 µM/L) and IGF-1 (10 ng/mL) for 24 hours. The ratios of ANNEXIN V positive cells were analyzed by statistical methods. Results show that GZD824 can facilitate apoptosis of the primary leukemia cells and the addition of cytokine IGF-1 can partly reverse the effect of GZD824 on apoptosis in the primary leukemia cells, demonstrating that the signal pathway activated by IGF-1 takes part when GZD824 is killing leukemia cells. Please refer to Fig. 17 for the experimental results.
6.3 After treating NALM6 cells with GZD824 of different concentrations (3 µM/L, 1 µM/L) for 24 hours, the cells were collected and lysed. With immunoblotting method, anti-phospho-AKT, AKT and IRS1 antibodies were used to analyze the lysates. The results show that GZD824 can down-regulate the expression of phosphorylated AKT and IRS1 in leukemia cells, demonstrating that GZD824 kills leukemia cells also by suppressing PI3K/AKT signal pathway.

Please refer to Fig. 18 for the experimental results.

6.4 The primary pre-B ALL cells treated with GZD824 are detected with Western blot method. Cells were respectively co-cultured with DMSO, GZD824 (1 µM), IGF-1 (10 ng/mL), or GZD824 (1 µM) and IGF-1 (10 ng/mL) for 24 hours. The cells were collected and lysed. The lysates were analyzed with phospho-AKT, AKT and IRS1 antibodies.

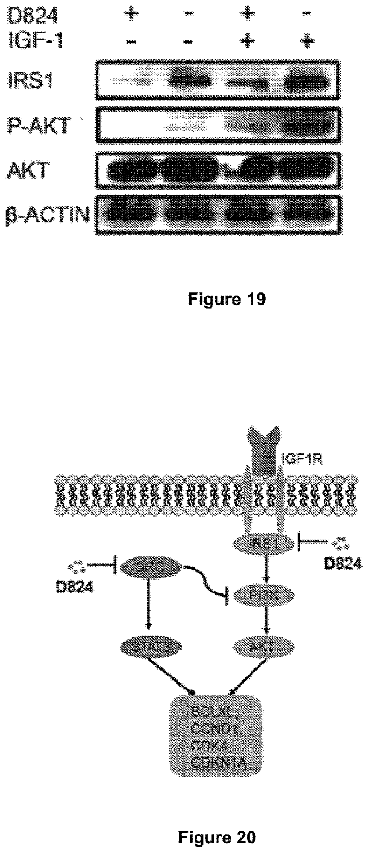

GZD824 kills the leukemia cells by suppressing two signal pathways, i.e. SRC/STAT3 and PI3K/AKT, and may be well applied in the treatment for Ph-ALL patients. Please refer to Fig. 20 for the results.

Each technical feature in the above-mentioned examples can be randomly combined. For consciences, not all possible combinations of various technical features in the above examples are described herein.

The above-mentioned examples merely provide several embodiments in the present invention with specific and detailed description, and they should not be regarded as limitations to the scope of protection of the 37398218-1 present invention. Therefore, the scope of protection of the present patent should be determined based on the claims.

## Claims

1. The compound 3-((1H-pyrazol[3,4-b]pyridine-5-substituted)ethinyl)-4- methyl-N-(4-((4-methylpiperazine-1-substituted)methyl)-3-(trifluoromethyl)phenyl)benzamide or a pharmaceutically acceptable salt thereof for use in a method of treating Ph-negative precursor B-cell lymphoblastic leukemia.

2. The compound for use in a method according to claim 1 wherein the compound enhances apoptosis of the Ph-negative precursor B-cell lymphoblastic leukemia cells.

3. The compound for use in a method according to claim 1, wherein the compound suppresses the cell cycle at a G0/G1 phase.

4. The compound for use in a method according to claim 1 wherein the compound does not kill normal B-cells.

5. The compound for use in a method according to claim 1 wherein the compound up-regulates expression of the CCKN1A gene.

6. The compound for use in a method according to claim 1 wherein the compound down-regulates the BXCL-XL, CCND1 and/or CDK4 genes.

7. The compound for use in a method according to claim 1 wherein the compound kills the Ph-negative precursor B-cell lymphoblastic leukemia cells via the SRC/STAT3 signal pathway.

8. The compound for use in a method according to claim 1 wherein the compound kills the Ph-negative precursor B-cell lymphoblastic leukemia cells by suppressing PI3K/AKT signal pathway.

## Patentansprüche

1. Verbindung 3-((1H-Pyrazol[3,4-b]pyridin-5-substituiert)ethinyl)-4-methyl-N-(4-((4-methylpiperazin-1-substituiert)methyl)-3-(trifluormethyl)phenyl)benzamid oder pharmazeutisch annehmbares Salz davon zur Verwendung in einem Verfahren zur Behandlung von Ph-negativer Vorläufer-B-Zell-lymphoblastischer Leukämie.

2. Verbindung zur Verwendung in einem Verfahren nach Anspruch 1, wobei die Verbindung eine Apoptose der Ph-negativen Vorläufer-B-Zell-lymphoblastischen Leukämiezellen verstärkt.

3. Verbindung zur Verwendung in einem Verfahren nach Anspruch 1, wobei die Verbindung den Zellzyklus in einer G0/G1-Phase unterdrückt.

4. Verbindung zur Verwendung in einem Verfahren nach Anspruch 1, wobei die Verbindung normale B-Zellen nicht abtötet.

5. Verbindung zur Verwendung in einem Verfahren nach Anspruch 1, wobei die Verbindung eine Expression des CCKN1A-Gens hochreguliert.

6. Verbindung zur Verwendung in einem Verfahren nach Anspruch 1, wobei die Verbindung das BXCL-XL-, das CCND1- und/oder das CDK4-Gen herunterreguliert.

7. Verbindung zur Verwendung in einem Verfahren nach Anspruch 1, wobei die Verbindung die Ph-negativen Vorläufer-B-Zell-lymphoblastischen Leukämiezellen über den SRC/STAT3-Signalweg abtötet.

8. Verbindung zur Verwendung in einem Verfahren nach Anspruch 1, wobei die Verbindung die Ph-negativen Vorläufer-B-Zell-lymphoblastischen Leukämiezellen durch Unterdrücken des PI3K/AKT-Signalwegs abtötet.

## Revendications

1. Composé 3-((1H-pyrazol[3,4-b]pyridine-5-substitué)éthinyl)-4-méthyl-N-(4-((4-méthylpipérazine-1-substitué)méthyl)-3-(trifluorométhyl)phényl)benzamide ou un sel pharmaceutiquement acceptable de celui-ci pour utilisation dans un procédé de traitement de la leucémie lymphoblastique à cellules B à précurseur Ph-négatif.

2. Composé pour utilisation dans un procédé selon la revendication 1, le composé favorisant l'apoptose des cellules de leucémie lymphoblastique à cellules B à précurseur Ph-négatif.

3. Composé pour utilisation dans un procédé selon la revendication 1, le composé supprimant le cycle cellulaire à une phase G0/G1.

4. Composé pour utilisation dans un procédé selon la revendication 1, le composé ne tuant pas les cellules B normales.

5. Composé pour utilisation dans un procédé selon la revendication 1, le composé régulant à la hausse l'expression du gène CCKN1A.

6. Composé pour utilisation dans un procédé selon la revendication 1, le composé régulant à la baisse les gènes BXCL-XL, CCND1 et/ou CDK4.

7. Composé pour utilisation dans un procédé selon la revendication 1, le composé tuant les cellules de leucémie lymphoblastique à cellules B à précurseur Ph-négatif via la voie de signalisation SRC/STAT3.

8. Composé pour utilisation dans un procédé selon la revendication 1, le composé tuant les cellules de leucémie lymphoblastique à cellules B à précurseur Ph-négatif en supprimant la voie de signalisation PI3K/AKT.
